# EUROPEAN PATENT SPECIFICATION

(11) **EP 3 247 320 B1**
(45) Date of publication and mention of the grant of the patent: **12.02.2020**
(21) Application number: 15804310.9
(22) Date of filing: 02.12.2015
(51) Int. Cl.: A61F 13/42, A61F 13/15

(54) **A METHOD FOR FASTENING AN ELECTRONIC UNIT TO A NAPPY**
VERFAHREN ZUR BEFESTIGUNG EINER ELEKTRONISCHEN EINHEIT AN EINER WINDEL
PROCÉDÉ PERMETTANT DE FIXER UNE UNITÉ ÉLECTRONIQUE À UNE COUCHE-CULOTTE

(30) Priority: 19.01.2015 DK 201570024
(43) Date of publication of application: 29.11.2017
(73) Proprietor: Suma Care ApS, 5000 Odense C (DK)
(72) Inventor: HANSEN, Martin Ettrup, 5300 Kerteminde (DK)
(74) Representative: Høiberg P/S
(86) International application number: PCT/DK2015/050374
(87) International publication number: WO 2016/116106

(56) References cited:
- WO-A1-2015/003712
- US-A1- 2007 252 711
- US-A1- 2009 315 728
- US-A1- 2013 110 063

## Description

The present invention relates to a method for fastening an electronic unit comprising, for instance, a data logger and/or a data transmitter to a nappy, which nappy is provided with one or more sensor elements for detecting the presence of liquid, and potentially also a measure of the amount thereof, within the nappy. Furthermore, the invention relates to a sensor element and an electronic unit for being used in such a method. The invention further relates to a sensor element in the form of a sensor strip, and an electronic unit, for being used in the method for fastening an electronic to a nappy. The invention further relates to a method for detecting the presence of liquid within a nappy.

### Background of the invention

During recent years, several sensor systems to be used for measuring the presence and, in some cases, the amount of liquid in nappies have been marketed. Such systems are typically characterised by emission of an alarm either as soon as a liquid is present on the sensor, a so-called instant alarm, or when a certain amount of liquid has built up within the nappy.

A sensor element is typically placed within the nappy at the time of production and an electronic unit comprising an electric power source for the sensor and electronics for receiving and logging data relating to the presence and/or amount of liquid within the nappy as well as for transmitting those data to an external monitoring unit of some kind.

In general, such a sensor system is designed for and at least partly integrated within a specific type of nappy. Especially the different solutions used for the mechanical fastening of the electronic unit to the nappy makes it difficult, if not even impossible, to reuse a given sensor system with another type of nappy than the one, it was originally designed for.

US 2009/0315728 A1 discloses a method of reducing sensor corrosion in an absorbent article. A wetness sensor is integrated into an absorbent article. The method includes a sequence of steps including applying voltage to a first conductor of the wetness sensor, measuring the potential across the conductors, and discontinuing the voltage to the first conductor.

US 2013/0110063 A1 discloses an absorbent article incorporating a non-contact electronic sensor array that may indicate the presence of a body exudate. The document discloses a complete gas sensor package with a housing, which is attached to an edge of a nappy.

US 2007/0252711 A1 discloses a monitor, for use with an absorbent article having a wetness sensor, including a power management system. The sensor is integrated in the absorbent article.

### Brief description of the invention

It is an object of the present invention to overcome the above-mentioned disadvantage of sensor systems known in the art by providing a flexible sensor system solution, which can be used with almost any type of nappy.

The present invention relates, in a first aspect, to a method for fastening an electronic unit to a nappy, said method comprising the steps of: providing a sensor element (6) comprising a contact area (7) arranged between two attachment parts which can be fastened to the nappy (10), said contact area (7) having a first side and a second side opposite the first side; fastening the attachment parts of the sensor element (6) to the surface of the nappy (10), preferably by means of an adhesive, in a fixed position in relation to the nappy (10), leaving the contact area (7) of the sensor element (6) free from being fastened directly to the surface of the nappy; providing an electronic unit (1) comprising a first part (3) and a second part (2); placing the first part (3) of the electronic unit (1) between the contact area (7) and the surface of the nappy (10) in close contact with the first side of the contact area (7); and placing the second part (2) of the electronic unit in close contact with the second side of the contact area (7) opposite the first side thereof, so that the contact area (7) of the sensor element (6) is wedged in between the first and the second part of the electronic unit (1), whereby the electronic unit (1) is attached to and held in a fixed position in relation to the sensor element (6) and, thereby, also to the nappy (1).

This configuration is advantageous in that it is a very flexible solution, which is also easily handled by the user. It can be used for substantially all different types of nappies. If only the sensor element is produced in a suitable size (typically meaning with a suitable length) for a given nappy, it can be fastened to the nappy, for instance using an adhesive placed on one side of the sensor leaving out the contact area.

In an embodiment of the invention, the first and second parts of the electronic unit are constituted by a body part and a lid part thereof, respectively, the lid part being hinged to the body part.

If the two parts of the electronic unit are hinged to each other, it is relatively easy to make a mechanically stable electronic unit, whether it is open or it is closed around the sensor element.

In an embodiment of the invention, the contact area is arranged between two other parts of the sensor element, which two parts are both fastened directly to the surface of the nappy.

If the sensor element is fastened to the nappy on both sides of the electronic unit, the fixation of the electronic unit is more stable.

In an embodiment of the invention, the end of the sensor element, which is nearest the contact area is arranged to be folded around the edge of the nappy and to be fastened to both the inner surface and the outer surface of the nappy.

If the sensor element is fastened to both sides of the nappy, the mechanically stability of the fastening increases.

In an embodiment of the invention, the position of the electronic unit is fixed in relation to the sensor element at least partly by means of one or more guide pins of the electronic unit fitting into correspondingly arranged, preferably through-going, holes in the contact area of the sensor strip.

This is a simple and reliable way of ensuring that the electronic unit does not move relatively to the sensor element.

In an embodiment of the invention, the sensor elements is arranged to be used for detecting the presence of liquid, and potentially also a measure of the amount thereof, within the nappy.

It is advantageously to use the method with such types of sensors, because they are widely known in many different types of nappies.

In an embodiment of the invention, the electronic unit comprises a data logger and/or a data transmitter arranged to provide the sensor element with one or more predefined electric signals, to receive and log one or more electric values from the sensor element in response thereto, and/or to transmit the logged values or a representation thereof wirelessly to an external receiver.

It is advantageous to gather all the electric and electronic parts of a sensor system within the same electronic unit in order to reduce the number of parts of the system.

In an embodiment of the invention, electric contact between one or more electronic contact points of the electronic unit and one or more electrodes of the sensor element is obtained in the contact area of the sensor element through one or more access holes arranged within the contact area.

This solution is advantageous in that it eliminates the need for an electric connector part to be mounted on the sensor element, which significantly reduces the production costs of the sensor element.

A further aspect of the invention relates to a sensor element in the form of a sensor strip (6) for being used in a method as described above, said sensor element (6) comprising at least two electrodes, and a contact area (7) arranged between two attachment parts of the sensor strip (6), said two attachment parts comprising an adhesive part on both sides of the contact area (7) for fastening the sensor element to a nappy (10) in a fixed position in relation to the nappy, wherein the contact area (7) is arranged to be free from being fastened directly to the surface of the nappy and wherein the contact area (7) comprises one or more access holes (9) in connection with the at least two electrodes, said access holes (9) being arranged for receiving corresponding electronic contact points of an electronic unit.

A further aspect of the invention relates to an electronic unit for being used in a method as described above, said electronic unit comprising a body part (3) and a lid part (2), the lid part (2) being hinged to the body part (3), wherein said electronic unit (1) is configurable to wedge a contact area of a sensor strip for a nappy between the body part (3) and the lid part (2) in a closed configuration, the electronic unit (1) further comprising one or more electronic contact points (5), wherein the one or more electronic contact points (5) are in direct physical and electrical contact of electrodes of the sensor strip in the closed configuration, and wherein the body part (3) and a lid part (2) is adapted to hold the electronic unit (1) in a fixed position in relation to the sensor element in the closed configuration.

A further aspect of the invention relates to a method for detecting the presence of liquid within a nappy (10), the method comprising the steps of: providing a sensor element (6) as described above; providing an electronic unit (1) as described above according to claim 9; and using the sensor element (6) to detect the presence of liquid within the nappy (10).

### The figures

In the following, an exemplary embodiment of the invention is described in more detail with reference to the figures, of which
- Fig. 1: is a schematic view of an electronic unit attached to a sensor which, in turn, is attached to a nappy according to an embodiment of the invention,
- Fig. 2a: is a perspective view of an open electronic unit according to an embodiment of the invention,
- Fig. 2b: is a perspective view of the electronic when it is closed,
- Fig. 3a: is a schematic view of a part of a sensor according to an embodiment of the invention as seen from a first side,
- Fig. 3b: is a schematic view of the same part of the sensor as seen from the opposite side,
- Fig. 4a: is a schematic top view of an electronic unit attached to a sensor,
- Fig. 4b: is a schematic end view of an electronic unit attached to a sensor, and
- Fig. 4c: is a schematic side view of an electronic unit attached to a sensor.

### Detailed description

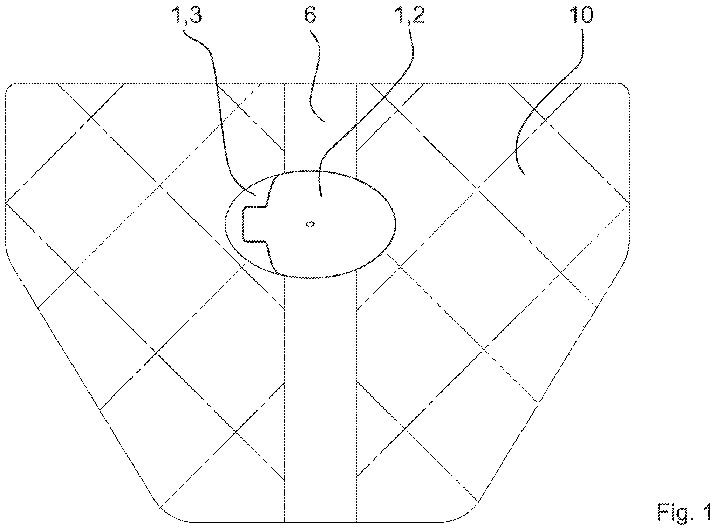

Fig. 1 is a schematic view of an electronic unit 1 attached to a sensor strip 6 which, in turn, is attached to a nappy 10 according to an embodiment of the invention. The sensor strip 6 comprises at least two electrodes (not shown), between which a voltage or a pattern of varying voltages can be applied by the electronic unit 1. Different electric values relating to the electrodes such as, for instance, resistance and/or capacitance between them can be registered by electronic unit 1. These electric values typically depend on the wetness of the nappy 10 and, therefore, the values registered by the electronic unit 1 can be used to estimate the amount of liquid accumulated within the nappy 10.

In the illustrated embodiment, the sensor strip 6 is fastened to a nappy 10 by means of a layer of adhesive or of double adhesive tape (not shown) placed on the side of the sensor strip 6, which faces the surface of the nappy 10. Basically, the only property of the sensor strip 6 to be adjusted in order to make it fit any given type of nappy 10 is the length of the sensor strip 6 in accordance with the desired position of the electronic unit 1 in relation to the given type of nappy 10. This property is easily adjusted during the production process and, therefore, systems using the sensor strip 6 are extremely flexible when it comes to the ability to be used with different types of nappies 10.

As can be seen from the figure, the electronic unit 1 comprises a first part in the form of a body part 3 and a second part in the form of a lid part 2 as will be discussed in further detail here below.

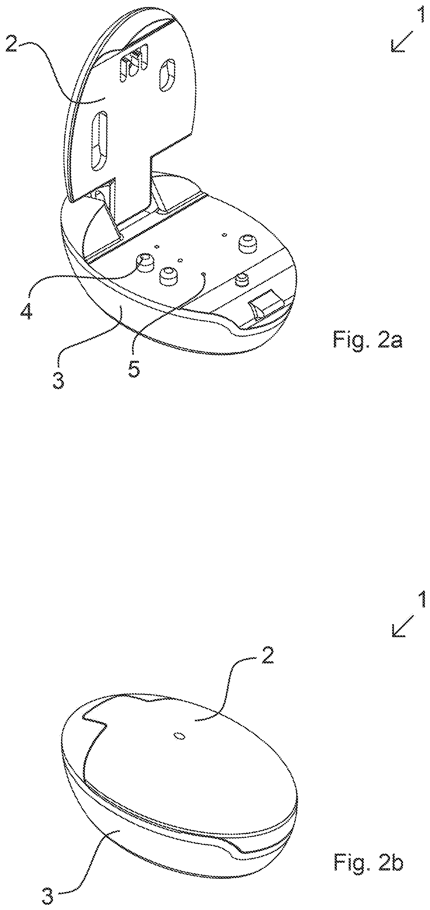

Figs. 2a and 2b are perspective views of the electronic unit 1 shown in Fig. 1 in an open and a closed configuration, respectively. In the shown embodiment, the body part 3 comprises three guide pins 4 arranged to pass through holes in the sensor strip 6 for holding the electronic unit 1 in a fixed position in relation to the sensor strip 6 when the electronic unit 1 is closed around the sensor strip 6 wedging the sensor strip 6 in between the body part 3 and the lid part 2 of the electronic unit 1 as described here below. Furthermore, Fig. 2a illustrates how the body part 3 is configured with a number of electronic contact points 5, through which the electronic unit 1 can be electrically connected to the electrodes of the sensor strip 6 for inducing voltages between the electrodes and for registering different electric values as mentioned above.

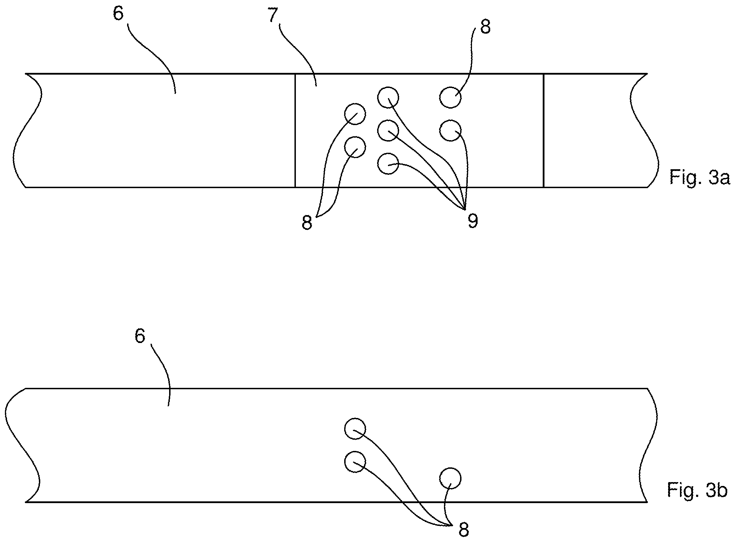

Figs. 3a and 3b illustrate schematically the two sides of a part of the sensor strip 6, respectively. A specific part of the sensor strip 6 is designed as a contact area 7 for being wedged in between the body part 3 and the lid part 2 of an electronic unit 1.

In the illustrated embodiment, the contact area 7 of the sensor strip 6 is penetrated by three through-going holes 8, the positions of which are arranged to correspond to the positions of the three guide pins 4 of the electronic unit 1 as described above. Thus, when the contact area 7 is placed within the electronic unit 1 and the electronic unit 1 is closed, the positions of the sensor strip 6 and the electronic unit 1 are fixed with respect to each other.

Furthermore, Fig. 3a illustrates how a number of access holes 9 to the electrodes (not shown) inside the sensor strip 6 are arranged within the contact area 7. The positions of these access holes 9 are arranged to correspond to the positions of the electronic contact points 5 of the electronic unit 1 described above. Thus, when the contact area 7, and thereby the sensor strip 6, is wedged in between the body part 3 and the lid part 2 of the electronic unit 1, the different electronic contact points 5 of the electronic unit 1 are in direct physical and electrical contact with the different electrodes within the sensor strip 6, respectively, through these access holes 9.

The electronic unit 1 is fastened indirectly to the nappy 10 by fastening it to the sensor strip 6, which, in turn, is fastened to the nappy 10 as described above. This is done by placing the body part 3 of the electronic unit 1 between the nappy 10 and the contact area 7 of the sensor strip 6 and, subsequently, closing the lid part 2 of the electronic unit 1 for wedging in the contact area 7 between the body part 3 and the lid part 2. In order for the contact area 7 not to attach to the nappy 10, either there is no adhesive or double adhesive tape on this part of the sensor strip 6 or the adhesive side facing the nappy 10 is covered by a layer of plastic or another suitable material.

In principle, the contact area 7 may be arranged at an end of the sensor strip 6. In this case, however, it may be necessary to mechanically stabilise the electronic unit 1 further, for instance by letting a piece of underwear or the like keep it in place or by placing hook-and-loop fastener components (commonly referred to as "Velcro") on the electronic unit 1 and on the surface of the nappy 10, respectively.

Preferably, therefore, the contact area 7 is arranged at a certain distance from the ends of the sensor strip 6 as illustrated in Figs. 3a and 3b so that there is an adhesive part of the sensor strip 6 on both sides of the contact area 7. This configuration ensures a more stable and reliable fastening of the electronic unit 1 to the nappy 1. As mentioned above, the adhesive part of the sensor strip 6 may continue over the edge of the nappy 10 to be adhered on both the inner and the outer surface of the nappy 10 for increasing the stability and reliability of the fastening even further.

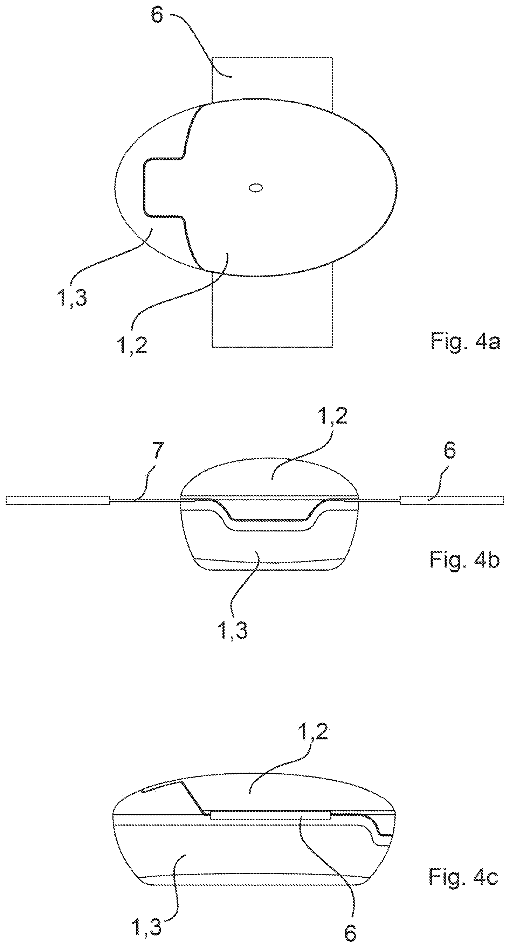

Figs. 4a-4c show schematically a top view, an end view and a side view, respectively of the electronic unit 1 attached to the sensor strip 6.

### List of reference numbers

- 1.: Electronic unit
- 2.: Lid part of electronic unit
- 3.: Body part of electronic unit
- 4.: Guide pin
- 5.: Electronic contact point
- 6.: Sensor strip
- 7.: Contact area of sensor strip
- 8.: Through-going guide hole of sensor strip
- 9.: Access hole for electrode in sensor strip
- 10.: Nappy

## Claims

1. A method for fastening an electronic unit (1) to a nappy (10), said method comprising the steps of
providing a sensor element (6) comprising a contact area (7) arranged between two attachment parts which can be fastened to the nappy (10), said contact area (7) having a first side and a second side opposite the first side,
fastening the attachment parts of the sensor element (6) to the surface of the nappy (10), preferably by means of an adhesive, in a fixed position in relation to the nappy (10), leaving the contact area (7) of the sensor element (6) free from being fastened directly to the surface of the nappy,
providing an electronic unit (1) comprising a first part (3) and a second part (2), placing the first part (3) of the electronic unit (1) between the contact area (7) and the surface of the nappy (10) in close contact with the first side of the contact area (7), and
placing the second part (2) of the electronic unit in close contact with the second side of the contact area (7) opposite the first side thereof, so that the contact area (7) of the sensor element (6) is wedged in between the first and the second part of the electronic unit (1), whereby the electronic unit (1) is attached to and held in a fixed position in relation to the sensor element (6) and, thereby, also to the nappy (1).

2. The method according to claim 1, wherein the first and second parts of the electronic unit (1) are constituted by a body part (3) and a lid part (2) thereof, respectively, the lid part (2) being hinged to the body part (3).

3. The method according to claim 1, wherein the end of the sensor element (6), which is nearest the contact area (7) is arranged to be folded around the edge of the nappy (10) and to be fastened to both an inner surface and an outer surface of the nappy.

4. The method according to any of the preceding claims, wherein the position of the electronic unit (1) is fixed in relation to the sensor element (6) at least partly by means of one or more guide pins (4) of the electronic unit fitting into correspondingly arranged, preferably through-going, holes (8) in the contact area of the sensor element (6).

5. The method according to any of the preceding claims, wherein the sensor element (6) is arranged to be used for detecting the presence of liquid, and potentially also a measure of the amount thereof, within the nappy (10).

6. The method according to any of the preceding claims, wherein the electronic unit (1) comprises a data logger and/or a data transmitter arranged
to provide the sensor element (6) with one or more predefined electric signals,
to receive and log one or more electric values from the sensor element (6) in response thereto, and/or
to transmit the logged values or a representation thereof wirelessly to an external receiver.

7. The method according to any of the preceding claims, wherein electric contact between one or more electronic contact points (5) of the electronic unit and one or more electrodes of the sensor element (6) is obtained in the contact area (7) of the sensor element through one or more access holes (9) arranged within the contact area.

8. A sensor element (6) in the form of a sensor strip (6) for being used in a method according to any of the preceding claims, said sensor element (6) comprising at least two electrodes, and a contact area (7) arranged between two attachment parts of the sensor strip (6), said two attachment parts comprising an adhesive part on both sides of the contact area (7) for fastening the sensor element to a nappy (10) in a fixed position in relation to the nappy, wherein the contact area (7) is arranged to be free from being fastened directly to the surface of the nappy and wherein the contact area (7) comprises one or more access holes (9) in connection with the at least two electrodes, said access holes (9) being arranged for receiving corresponding electronic contact points of an electronic unit.

9. An electronic unit (1) for being used in a method according to any of claims 1-7, said electronic unit comprising a body part (3) and a lid part (2), the lid part (2) being hinged to the body part (3), wherein said electronic unit (1) is configurable to wedge a contact area of a sensor strip for a nappy between the body part (3) and the lid part (2) in a closed configuration, the electronic unit (1) further comprising one or more electronic contact points (5), wherein the one or more electronic contact points (5) are in direct physical and electrical contact of electrodes of the sensor strip in the closed configuration, and wherein the body part (3) and a lid part (2) is adapted to hold the electronic unit (1) in a fixed position in relation to the sensor element in the closed configuration.

10. A method for detecting the presence of liquid within a nappy (10), the method comprising the steps of:
providing a sensor element (6) according to claim 8;
providing an electronic unit (1) according to claim 9;
using the sensor element (6) to detect the presence of liquid within the nappy (10).

## Patentansprüche

1. Verfahren zur Befestigung einer elektronischen Einheit (1) an einer Windel (10), wobei das Verfahren folgende Schritte umfasst:
Bereitstellen eines Sensorelements (6), das einen Kontaktbereich (7) umfasst, der zwischen zwei Befestigungsteilen angeordnet ist, die an der Windel (10) angebracht werden können, wobei der Kontaktbereich (7) eine erste Seite und eine zweite Seite, die der ersten Seite gegenüberliegt, aufweist,
Anbringen der Befestigungsteile des Sensorelements (6) an der Oberfläche der Windel (10), vorzugsweise durch einen Klebstoff, in einer fixen Position in Bezug auf die Windel (10), wobei der Kontaktbereich (7) des Sensorelements (6) nicht direkt an der Oberfläche der Windel angebracht wird,
Bereitstellen einer elektronischen Einheit (1), die ein erstes Teil (3) und ein zweites Teil (2) umfasst,
Platzieren des ersten Teils (3) der elektronischen Einheit (1) zwischen dem Kontaktbereich (7) und der Oberfläche der Windel (10) in engem Kontakt mit der ersten Seite des Kontaktbereichs (7), und
Platzieren des zweiten Teils (2) der elektronischen Einheit in engem Kontakt mit der zweiten Seite des Kontaktbereichs (7) gegenüber von dessen erster Seite, sodass der Kontaktbereich (7) des Sensorelements (6) zwischen dem ersten und dem zweiten Teil der elektronischen Einheit (1) eingeklemmt ist, wobei die elektronische Einheit (1) in einer fixen Position in Bezug auf das Sensorelement (6), und somit auch auf die Windel (1), befestigt und gehalten wird.

2. Verfahren nach Anspruch 1, wobei das erste und das zweite Teil der elektronischen Einheit (1) aus einem Körperteil (3) bzw. einem Deckelteil (2) davon gebildet werden, wobei das Deckelteil (2) an dem Körperteil (3) angelenkt ist.

3. Verfahren nach Anspruch 1, wobei das Ende des Sensorelements (6), das dem Kontaktbereich (7) am nächsten ist, so angeordnet ist, dass es um den Rand der Windel (10) geklappt und sowohl an einer inneren Oberfläche als auch an einer äußeren Oberfläche der Windel angebracht werden kann.

4. Verfahren nach einem der vorhergehenden Ansprüche, wobei die Position der elektronischen Einheit (1) in Bezug auf das Sensorelement (6) mindestens teilweise durch einen oder mehrere Führungsstifte (4) der elektronischen Einheit, die in entsprechend angeordnete, vorzugsweise durchgehende, Löcher (8) in dem Kontaktbereich des Sensorelements (6) passen, fixiert ist.

5. Verfahren nach einem der vorhergehenden Ansprüche, wobei das Sensorelement (6) so angeordnet ist, dass es zum Erkennen des Vorhandenseins von Flüssigkeit in der Windel (10), und potentiell auch zu einem Messen von der Menge der Flüssigkeit, verwendet werden kann.

6. Verfahren nach einem der vorhergehenden Ansprüche, wobei die elektronische Einheit (1) einen Datenaufzeichner und/oder einen Datensender umfasst, die so angeordnet sind, dass sie:
dem Sensorelement (6) ein oder mehrere vorbestimmte elektrische Signale bereitstellen,
einen oder mehrere elektrische Werte von dem Sensorelement (6) in Reaktion darauf empfangen und aufzeichnen, und/oder
die aufgezeichneten Werte oder eine Repräsentation davon kabellos an einen externen Empfänger senden.

7. Verfahren nach einem der vorhergehenden Ansprüche, wobei der elektrische Kontakt zwischen einem oder mehreren elektronischen Kontaktpunkten (5) der elektronischen Einheit und einer oder mehrerer Elektroden des Sensorelements (6) in dem Kontaktbereich (7) des Sensorelements durch ein oder mehrere Zugangslöcher (9) hergestellt wird, die in dem Kontaktbereich angeordnet sind.

8. Sensorelement (6) in der Form eines Sensorbands (6) zum Verwenden in einem Verfahren nach einem der vorhergehenden Ansprüche, wobei das Sensorelement (6) mindestens zwei Elektroden und einen Kontaktbereich (7), der zwischen zwei Befestigungsteilen des Sensorbands (6) angeordnet ist, umfasst, wobei die zwei Befestigungsteile ein Klebstoffteil auf beiden Seiten des Kontaktbereichs (7) umfassen, um das Sensorelement in einer fixen Position in Bezug auf die Windel an einer Windel (10) anzubringen, wobei der Kontaktbereich (7) so angeordnet ist, dass er nicht direkt an der Oberfläche der Windel angebracht werden kann, und wobei der Kontaktbereich (7) ein oder mehrere Zugangslöcher (9) in Verbindung mit den mindestens zwei Elektroden umfasst, wobei die Zugangslöcher (9) so angeordnet sind, dass sie entsprechende elektronische Kontaktpunkte einer elektronischen Einheit empfangen.

9. Elektronische Einheit (1) zum Verwenden in einem Verfahren nach einem der Ansprüche 1-7, wobei die elektronische Einheit ein Körperteil (3) und ein Deckelteil (2) umfasst, wobei das Deckelteil (2) an dem Körperteil (3) angelenkt ist, wobei die elektronische Einheit (1) dazu konfigurierbar ist, einen Kontaktbereich eines Sensorbands für eine Windel zwischen dem Körperteil (3) und dem Deckelteil (2) in geschlossener Konfiguration einzuklemmen, wobei die elektronische Einheit (1) ferner einen oder mehrere elektronische Kontaktpunkte (5) umfasst, wobei der eine oder die mehreren elektronischen Kontaktpunkte (5) in der geschlossenen Konfiguration in direktem physischem und elektrischem Kontakt mit Elektroden des Sensorbands sind, und wobei das Körperteil (3) und ein Deckelteil (2) dazu ausgelegt sind, in der geschlossenen Konfiguration die elektronische Einheit (1) in einer fixen Position in Bezug auf das Sensorelement zu halten.

10. Verfahren zum Erkennen des Vorhandenseins von Flüssigkeit in einer Windel (10), wobei das Verfahren folgende Schritte umfasst:
Bereitstellen eines Sensorelements (6) nach Anspruch 8;
Bereitstellen einer elektronischen Einheit (1) nach Anspruch 9;
Verwenden des Sensorelements (6), um das Vorhandensein von Flüssigkeit in der Windel (10) zu erkennen.

## Revendications

1. Procédé pour attacher une unité électronique (1) à une couche (10), ledit procédé comprenant les étapes consistant à :
fournir un élément de détection (6) comprenant une zone de contact (7) disposée entre deux parties de fixation qui peuvent être attachées à la couche (10), ladite zone de contact (7) présentant un premier côté et un second côté en face du premier côté,
attacher les parties de fixation de l'élément de détection (6) à la surface de la couche (10), de préférence au moyen d'un adhésif, dans une position fixe par rapport à la couche (10), laissant la zone de contact (7) de l'élément de détection (6) dans l'incapacité d'être attachée directement à la surface de la couche,
fournir une unité électronique (1) comprenant une première partie (3) et une seconde partie (2),
placer la première partie (3) de l'unité électronique (1) entre la zone de contact (7) et la surface de la couche (10) en contact étroit avec le premier côté de la zone de contact (7), et
placer la seconde partie (2) de l'unité électronique en contact étroit avec le second côté de la zone de contact (7) en face du premier côté de celle-ci, de sorte que la zone de contact (7) de l'élément de détection (6) est coincée entre la première et la seconde partie de l'unité électronique (1), moyennant quoi l'unité électronique (1) est fixée et maintenue dans une position fixe par rapport à l'élément de détection (6) et, ainsi, également à la couche (1).

2. Procédé selon la revendication 1, dans lequel les première et seconde parties de l'unité électronique (1) sont constituées par une partie corps (3) et une partie couvercle (2) de celle-ci, respectivement, la partie couvercle (2) étant articulée sur la partie corps (3).

3. Procédé selon la revendication 1, dans lequel l'extrémité de l'élément de détection (6), qui est la plus proche de la zone de contact (7), est agencée pour être pliée autour du bord de la couche (10) et pour être attachée à la fois à une surface interne et à une surface externe de la couche.

4. Procédé selon l'une quelconque des revendications précédentes, dans lequel la position de l'unité électronique (1) est fixe par rapport à l'élément de détection (6) au moins partiellement au moyen d'une ou plusieurs goupilles de guidage (4) de l'unité électronique s'ajustant dans des trous (8) agencés de manière correspondante, de préférence traversants, dans la zone de contact de l'élément de détection(6).

5. Procédé selon l'une quelconque des revendications précédentes, dans lequel l'élément de détection (6) est agencé pour être utilisé pour détecter la présence de liquide, et potentiellement également une mesure de la quantité de celui-ci, au sein de la couche (10).

6. Procédé selon l'une quelconque des revendications précédentes, dans lequel l'unité électronique (1) comprend un enregistreur de données et/ou un transmetteur de données agencés
pour fournir à l'élément de détection (6) un ou plusieurs signaux électriques prédéfinis,
pour recevoir et enregistrer une ou plusieurs valeurs électriques en provenance de l'élément de détection (6) en réponse à celui-ci, et/ou
pour transmettre les valeurs enregistrées ou une représentation de celles-ci sans fil à un récepteur externe.

7. Procédé selon l'une quelconque des revendications précédentes, dans lequel un contact électrique entre deux ou plus de deux points de contact électronique (5) de l'unité électronique et une ou plusieurs électrodes de l'élément de détection (6) est obtenu dans la zone de contact (7) de l'élément de détection à travers un ou plusieurs trous d'accès (9) agencés au sein de la zone de contact.

8. Élément de détection (6) sous la forme d'une bande de détection (6) pour être utilisé dans un procédé selon l'une quelconque des revendications précédentes, ledit élément de détection (6) comprenant au moins deux électrodes, et une zone de contact (7) agencée entre deux parties de fixation de la bande de détection (6), lesdites deux parties de fixation comprenant une partie adhésive sur les deux côtés de la zone de contact (7) pour attacher l'élément de détection à une couche (10) dans une position fixe par rapport à la couche, dans lequel la zone de contact (7) est agencée pour être dans l'incapacité de pouvoir être attachée directement à la surface de la couche et dans lequel la zone de contact (7) comprend un ou plusieurs trous d'accès (9) en relation avec les au moins deux électrodes, lesdits trous d'accès (9) étant agencés pour recevoir des points de contact électronique correspondants d'une unité électronique.

9. Unité électronique (1) pour être utilisée dans un procédé selon l'une quelconque des revendications 1 à 7, ladite unité électronique comprenant une partie corps (3) et une partie couvercle (2), la partie couvercle (2) étant articulée sur la partie corps (3), dans laquelle ladite unité électronique (1) est configurable pour coincer une zone de contact d'une bande de détection pour une couche entre la partie corps (3) et la partie couvercle (2) dans une configuration fermée, l'unité électronique (1) comprenant en outre un ou plusieurs points de contact électronique (5), dans lequel les un ou plusieurs points de contact électronique (5) sont en contact physique et électrique direct d'électrodes de la bande de détection dans la configuration fermée, et dans laquelle la partie corps (3) et une partie couvercle (2) sont adaptées pour maintenir l'unité électronique (1) dans une position fixe par rapport à l'élément de détection dans la configuration fermée.

10. Procédé pour détecter la présence de liquide au sein d'une couche (10), le procédé comprenant les étapes consistant à :
fournir un élément de détection (6) selon la revendication 8 ;
fournir une unité électronique (1) selon la revendication 9 ;
utiliser l'élément de détection (6) pour déceler la présence de liquide au sein de la couche (10).
